# EUROPEAN PATENT SPECIFICATION

(11) **EP 1 780 671 B1**
(45) Date of publication and mention of the grant of the patent: **16.04.2014**
(21) Application number: 06022582.8
(22) Date of filing: 30.10.2006
(51) Int. Cl.: G06T 7/00, G06T 7/60

(54) **Image processing system and method for editing contours of a target object using multiple sectional images**
Bildverarbeitungssystem und -verfahren zum Editieren von Konturen eines Zielobjekts anhand mehrerer Schnittbilder
Système et procédé de traitement d'images pour l'édition de contours d'une cible à base de plusieurs images en coupe

(30) Priority: 01.11.2005 KR 20050103640
(43) Date of publication of application: 02.05.2007
(73) Proprietor: MEDISON CO., LTD., Kangwon-do 250-870 (KR)
(72) Inventor: Ahn, Chi Young Discusser & Medison Building, Seoul 135-280 (KR); Lee, Jin Yong Discusser & Medison Building, Seoul 135-280 (KR)
(74) Representative: Schmid, Wolfgang

(56) References cited:
- EP-A2- 1 083 443
- WO-A-01/01864
- VAN STRALEN ET AL: "Left Ventricular Volume Estimation in Cardiac Three-dimensional Ultrasound" ACADEMIC RADIOLOGY, RESTON, VA, US, vol. 12, no. 10, October 2005 (2005-10), pages 1241-1249, XP005117142 ISSN: 1076-6332

## Description

### FIELD OF THE INVENTION

The present invention generally relates to image processing systems, and more particularly to an image processing system and method for editing the contours of a target object by using multiple sectional images.

### BACKGROUND OF THE INVENTION

An image processing system, which is used for processing an image of a target object and displaying the processed image, has been widely used. As an example of such an image processing system, an image processing system for conducting ultrasound diagnosis (hereinafter referred to as an "ultrasound diagnostic system") will be described.

Generally, an ultrasound diagnostic system has become an important and popular diagnostic tool due to its wide range of applications. Specifically, due to its non-invasive and non-destructive nature, the ultrasound diagnostic system has been extensively used in the medical profession. Modem high-performance ultrasound diagnostic systems and techniques are commonly used to produce two or three-dimensional (2D or 3D) diagnostic images of a target object. The ultrasound diagnostic system generally uses a probe including an array transducer having a plurality of transducer elements to transmit and receive ultrasound signals. The ultrasound diagnostic system forms ultrasound images of the internal structures of the target object by electrically exciting the transducer elements to generate ultrasound pulses that travel into the target object. The ultrasound pulses produce ultrasound echoes since they are reflected from a discontinuous surface of acoustic impedance of the internal structure, which appears as discontinuities to the propagating ultrasound pulses. Various ultrasound echoes return to the array transducer and are converted into electrical signals, which are amplified and processed to produce ultrasound data for forming an image of the internal structure of the target object.

Especially, in a conventional ultrasound diagnostic system, sectional images are extracted from 3D ultrasound image data of the target object at every specified angle by rotating the 3D image data by specified angles. Then, the contours of the target object are automatically detected on the extracted sectional images and a 3D volume image of the target object is formed by using the sectional images including the contours to thereby measure a volume of the target object.

In order to more precisely measure the volume of the target object, the conventional ultrasound diagnostic system provides a function for editing the contours detected on the sectional images by the user through an input unit. That is, in the conventional ultrasound diagnostic system, a single sectional image extracted at a specified angle is displayed and the contour detected on the sectional image is edited by the user. After editing the contour, the next sectional image is displayed to edit the contour thereon.

However, since only one sectional image is displayed to edit the contour in the conventional ultrasound diagnostic system, the user cannot refer to the contours detected on other sectional images in contour editing. Further, it takes a long time to edit the contours detected on the sectional images.

EP 1 083 443 A2 discloses an ultrasonic image apparatus in which a diagnostic object is separated from the background to show the separated object in a three dimensional image. A contour of a target object is automatically separated and extracted and the extracted information is used to visualize the object in three dimension.

In WO 01/01864 there is described an intravascular ultrasound analysis system and method which determines luminal and medical-adventitial boundaries of a blood vessel. Ultrasonic data is acquired by a rotating transducer mounted to a tip of a catheter which is inserted into the blood vessel. An intravascular image is reconstructed from the ultrasound data. A boundary contour is generated based on the boundary points.

### SUMMARY OF THE INVENTION

The present invention provides an image processing system and method for editing the contours of a target object in multiple sectional images formed by slicing a 3D ultrasound image containing a target object image in a predetermined interval.

In accordance with one aspect of the present invention, there is provided an image processing system, according to claim 1.

In accordance with another aspect of the present invention, there is provided an image processing method, according to claim 4.

### BRIEF DESCRIPTION OF THE DRAWINGS

The above and other objects and features of the present invention will become apparent from the following description of an embodiment given in conjunction with the accompanying drawings, in which:
FIG. 1 is a block diagram showing an ultrasound diagnostic system constructed in accordance with one embodiment of the present invention;
FIG. 2 is a block diagram showing an image processor constructed in accordance with one embodiment of the present invention;
FIG 3 is a flowchart showing a volume measuring process using a contour editing function for editing contours of a target object in multiple sectional images in accordance with one embodiment of the present invention;
FIG 4 shows an exemplary rotation axis set in 3D ultrasound image data in accordance with one embodiment of the present invention;
FIG. 5 shows an example of the multiple sectional images in accordance with one embodiment of the present invention;
FIG 6 is a flowchart showing a contour editing process using the multiple sectional images in accordance with one embodiment of the present invention; and
FIGS. 7A to 7C show an example of contour editing using the multiple sectional images in accordance with one embodiment of the present invention.

### DETAILED DESCRIPTION OF THE PRESENT INVENTION

Hereinafter, one embodiment of the present invention will be described with reference to the accompanying drawings. The ultrasound diagnostic system will be described as an example of an image processing system, which is constructed in accordance with the present invention.

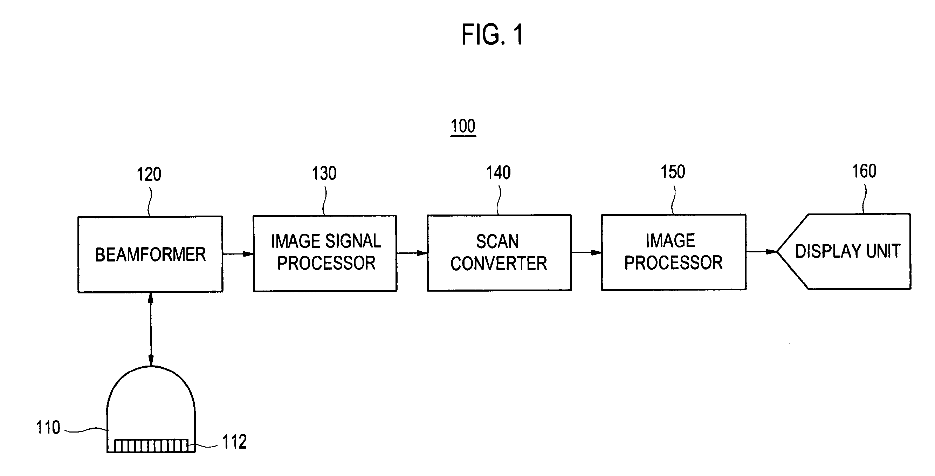

FIG. 1 is a block diagram showing an ultrasound diagnostic system constructed in accordance with one embodiment of the present invention. As shown in FIG. 1, the ultrasound diagnostic system 100 includes a probe 110, a beamformer 120, an image signal processor 130, a scan converter 140, an image processor 150 and a display unit 160. The image signal processor 130 and the image processor 150 may be provided as one processor.

The probe 110 includes a 1-dimensional or a 2-dimensional array transducer 112 including a plurality of transducer elements. The transmit signals, which are appropriately delayed in the beamformer 120 to form an ultrasound beam, are transmitted to the array transducer 112. Then, the focused ultrasound beam, which is produced in response to the transmit signals, is transmitted along a scan line set in a target object (not shown). The probe 110 receives ultrasound echo signals reflected from the target object and converts the ultrasound echo signals into electrical signals (hereinafter referred to as "receive signals''). The receive signals are transmitted to the beamformer 120.

The beamformer 120 provides delays of transmit signals to be transmitted to the array transducer 112 included in the probe 110 such that the ultrasound signals outputted from the array transducer 112 are focused on a focal point. Further, the beamformer 120 focuses the receive signals, which are received from the array transducer 112 included in the probe 110, in consideration of the delays with which the echo signals are arrived at each transducer element. It then outputs a focused receive beam representing the energy level of the ultrasound echo signals reflected from the focal point.

The image signal processor 130 (e.g., a digital signal processor (DSP)) performs an envelope detection for detecting the intensities of the focused receive signals to form ultrasound image data. That is, the image signal processor 130 forms ultrasound image data based on the receive focused signals acquired from each focal point and position information of a plurality of focal points on each scan line. The ultrasound image data include coordinates information of each focal point, angle information of each scan line and intensity information of the echo signals received at each focal point. The ultrasound image data may be 2D ultrasound data or 3D ultrasound data.

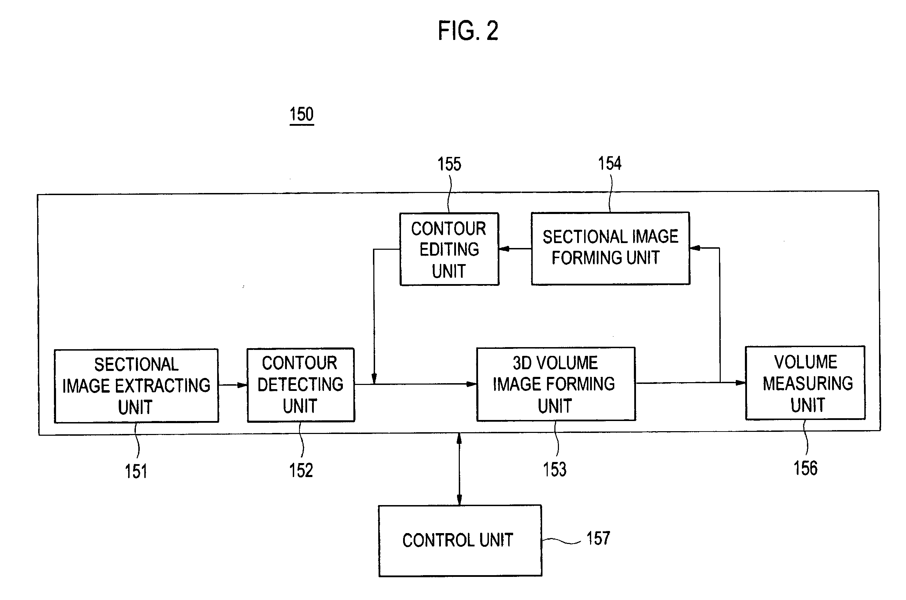

The scan converter 140 scan-converts the 3D ultrasound image data to a data format capable of being displayed on a screen of the display unit 160. As shown in FIG 2, the image processor 150 includes a sectional image extracting unit 151, a contour detecting unit 152, a 3D volume image forming unit 153, a sectional image forming unit 154, a contour editing unit 155, a volume measuring unit 156 and a control unit 157.

The sectional image extracting unit 151 sets a rotation axis passing through a center of 3D ultrasound image data provided from the scan converter 140. Then, the sectional image extracting unit 151 extracts sectional images at every specified angle (θ) from the 3D ultrasound image data by rotating it around the rotation axis.

The contour detecting unit 152 automatically detects the contours of the target object on the respective sectional images extracted from the 3D ultrasound image data by the sectional image extracting unit 151. The 3D volume image forming unit 153 forms a 3D volume image of the target object based on the contour of the target object provided from the contour detecting unit 152 and multiple sectional images provided from the contour editing unit 155.

The sectional image forming unit 154 sets a reference plane in the 3D ultrasound image and forms the multiple sectional images parallel to the reference plane by slicing the 3D ultrasound image in a predetermined interval. The contour editing unit 155 receives contour editing information upon the contours of the target object on the multiple sectional images through an input unit (not shown) from the user. For example, when a mouse is used as the input unit, the user can input contour editing information by drawing or dragging. It then edits the contours of the target object on the multiple sectional images based on the received editing information.

The volume measuring unit 156 measures the volume of the target object in the 3D volume image formed based on the contours of the target object, which are detected on the sectional images or edited on the multiple sectional images. The control unit 157 controls the overall operations of the image processor 150. For example, the control unit 157 checks whether the user selects contour editing and then operates each unit of the image processor 150 according to whether or not contour editing has been selected.

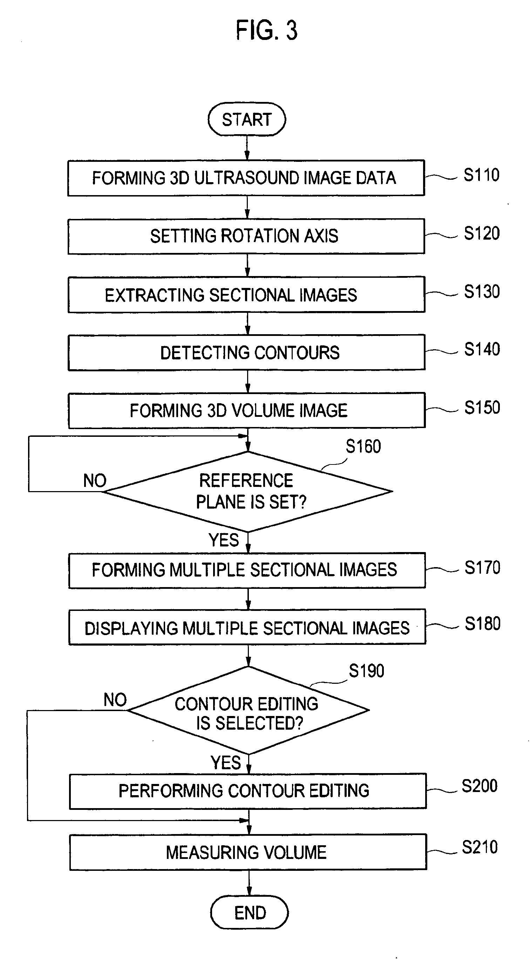

Hereinafter, the operations of the image processor will be described with reference to FIGS. 3 to 7C.

FIG. 3 is a flowchart showing a process for measuring the volume of the target object using a contour editing function for editing the contours of a target object in the multiple sectional images in accordance with one embodiment of the present invention.

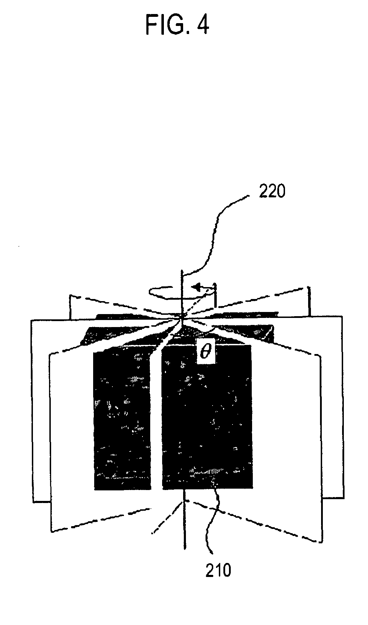

As shown in FIG. 3, at step S110, the 3D ultrasound image data are formed by the image signal processor 130 and the scan converter 140. At step S120, the sectional image extracting unit 151 of the image processor 150 sets a rotation axis passing through a center of the 3D ultrasound image data provided from the scan converter 140. Then, at step S130, the sectional image extracting unit 151 extracts sectional images at every specified angle θ from the 3D ultrasound image data 210 by rotating it around the rotation axis 220, as shown in FIG 4.

At step S140, the contour detecting unit 152 detects the contours of the target object on the extracted sectional images. Next, at step S150, the 3D volume image forming unit 153 forms a 3D volume image of the target object by using the detected contours of the target object.

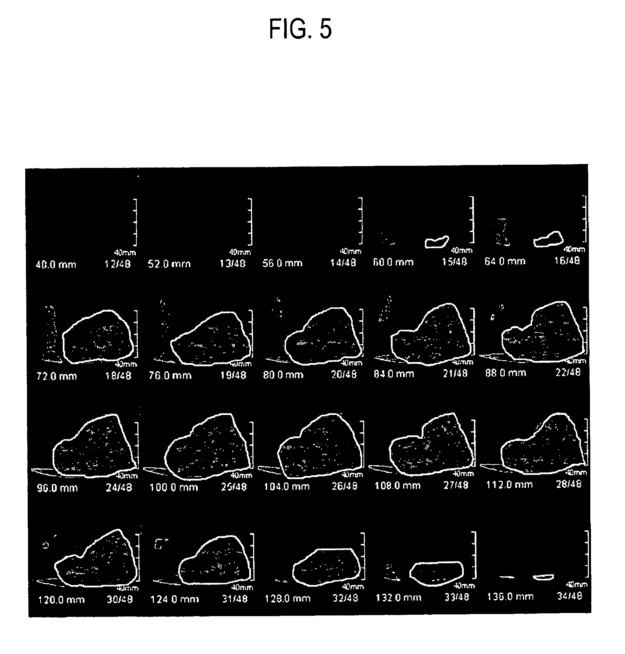

Next, at step S160. the control unit 157 checks whether the user sets a reference plane in the 3D ultrasound image to form multiple sectional images. If it is determined that the user has set the reference plane at step S160, then the process proceeds to step S170, wherein the sectional image forming unit 154 forms the multiple sectional images parallel to the reference plane by slicing the 3D ultrasound image in a predetermined interval. Then, at step S180, the multiple sectional images are displayed on the screen of the display unit 160, as shown in FIG 5

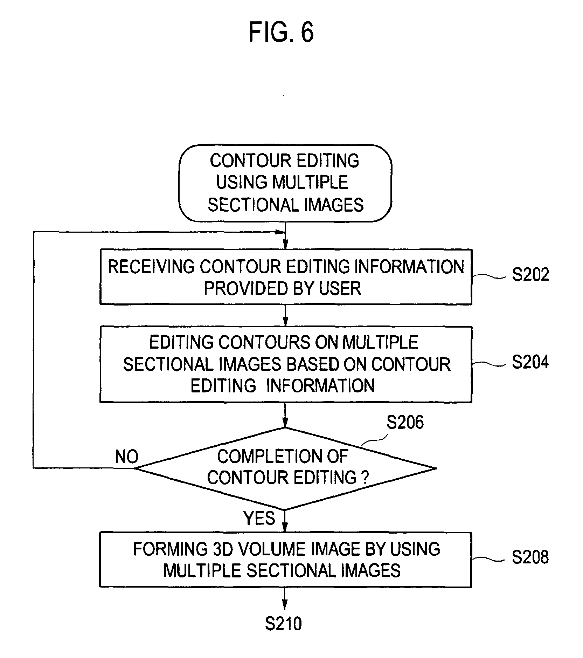

At step S190, the control unit 157 checks whether contour editing is selected by the user. If it is determined that contour editing has been selected at step SI90, then the process proceeds to step S200, wherein a contour editing process using the multiple sectional images is conducted. The contour editing process at step S200 will be described later with reference to FIGS. 6 to 7C.

If it is determined that contour editing has not been selected at step S190 or after the contour editing process at step S200, then the volume measuring unit 156 measures the volume of the target object in the 3D volume image provided from the 3D volume image forming unit 153 at step 5210. After the volume of the target object is measured, the image processor 150 completes the process.

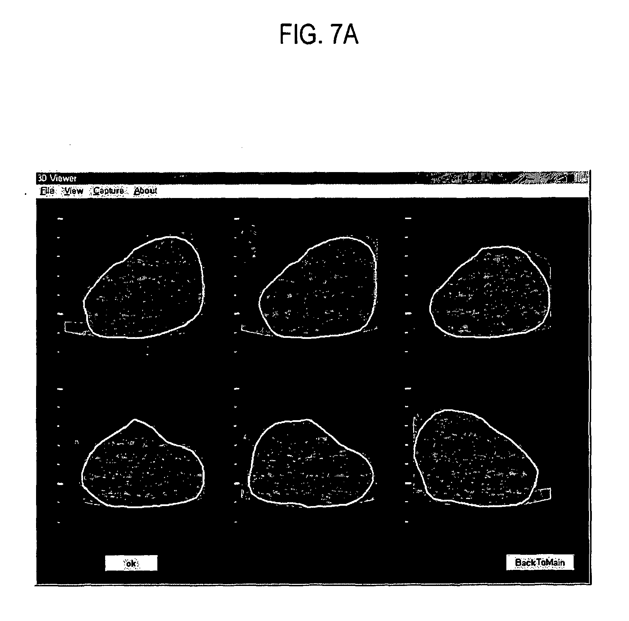

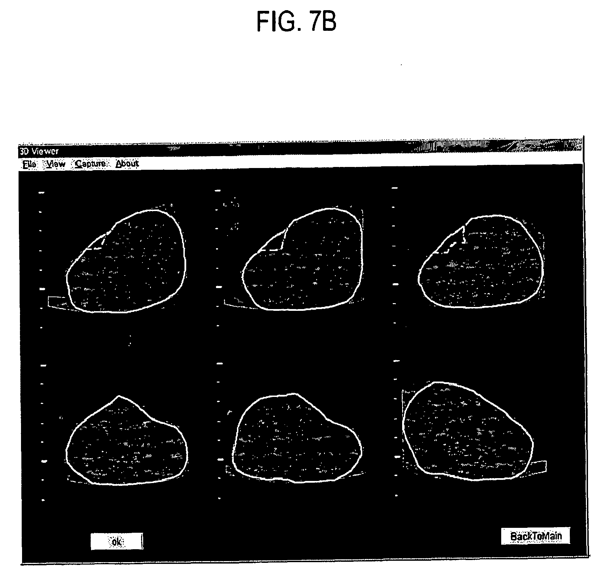

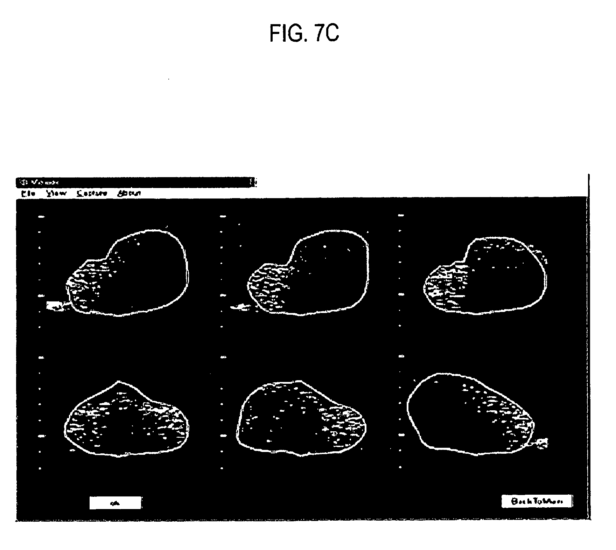

FIG 6 is a flowchart showing a contour editing process using the multiple sectional images in accordance with one embodiment of the present invention. As shown in FIG 6, if it is determined that the user has selected contour editing at step S190 shown in FIG 3, then the contour editing unit 155 receives contour editing information on the multiple sectional images provided by the user through the input unit at step S202. It then edits the contours on the multiple sectional images based on the received contour editing information step S204. Steps S202 and S204 are explained as follows with reference to FIGS. 7A to 7C.
(1) FIG 7A shows the multiple sectional images of the 3D ultrasound image formed by the sectional image forming unit 154, which are displayed on the screen of the display unit 160. In FIG 7A, solid lines represent the contours automatically detected by the contour detecting unit 152.
(2) The user edits the detected contours on the multiple sectional images through the input unit, as shown in FIG. 7B. For example, the user selects parts to be edited on the multiple sectional images through the input unit. As shown in FIG 7B, dashed dotted lines represent the contours edited by the user.
(3) The contour editing unit 155 edits the contours on the multiple sectional images based on the contour editing information provided by the user through the input unit, as shown in FIG. 7C.

Then, the control unit J 57 checks whether the user requests the completion of the contour editing process on the multiple sectional images at step S206. If it is determined that the user has not requested the completion of the contour editing process, then the process returns to step S202. However, if it is determined that the user has requested the completion of the contour editing process, then the 3D volume image forming unit 153 forms a 3D volume image by using the multiple sectional images at step S208 and then the process proceeds to step S210.

A 3D volume image of a target object is formed by using sectional images extracted at every specified angle by rotating the 3D ultrasound image data by specified angles. Then, the multiple sectional images are formed based on a reference plane in the 3D ultrasound image. However, in another example, the multiple sectional images can be formed directly from the 3D ultrasound image data based on the reference plane.

In accordance with the present invention, the contour of the target object is edited on the multiple sectional images formed by slicing the 3D ultrasound image. Accordingly, the user can easily observe how the contours are changed as the user edits them and the user can easily edit the contours.

While the present invention has been described and illustrated with respect to an embodiment of the invention, it will be apparent to those skilled in the art that variations and modifications are possible within the scope of the claims appended hereto.

## Claims

1. An image processing system, comprising:
a 3D image data forming unit for forming three-dimensional 3D image data based on input image signals;
a sectional image extracting unit (151) for extracting first sectional images from the 3D image data;
a contour detecting unit (152) for automatically detecting first contours of a target object on the first sectional images;
a sectional image forming unit (154) for setting a reference plane in the 3D image data and slicing the 3D image to form second sectional images based on the reference plane, wherein the second sectional images are parallel to the reference plane;
a contour editing unit (155) for editing second contours of the target object on the second sectional images based on contour editing information provided by a user; and
an image forming unit (153) for forming a 3D volume image based on the first contours provided from the contour detecting unit (152) and the second sectional images provided from the contour editing unit (155),
wherein the sectional image extracting unit (151) includes:
a unit for setting a rotation axis passing through a center of the 3D image data; and
a unit for extracting the first sectional images from the 3D image data at every specified angle by rotating the 3D image data around the rotation axis.

2. The image processing system of Claim 1, wherein the input image signals are ultrasound image signals.

3. The image processing system of Claim 1, further comprising an input unit for allowing the user to provide reference plane setting information and the contour editing information.

4. A method of processing an image, comprising the steps of:
a) forming 3D image data based on input image signals (S110);
b) extracting first sectional images from the 3D image data (S130);
c) automatically detecting first contours of a target object on the first sectional images (S140);
d) setting a reference plane in the 3D image data (S160) and slicing the 3D image to second sectional images based on the reference plane (S170), wherein the second sectional images are parallel to the reference plane; and
e) editing second contours of the target object on the second sectional images based on contour editing information provided by a user (S200), and
f) forming a 3D volume image based on the first contours and the second sectional images having the second contours (S210),
wherein step b) includes:
b1) setting a rotation axis passing through a center of the 3D image data; and
b2) extracting the first sectional images from the 3D image data at every specified angle by rotating the 3D image data around the rotation axis.

5. The method of Claim 4, wherein the input image signals are ultrasound image signals.

6. The method of Claim 4, wherein step d) includes:
d1) receiving reference plane setting information provided by a user; and
d2) forming the second sectional images from the 3D image data based on the reference plane setting information.

7. The method of Claim 4, wherein step e) includes:
e1) receiving the contour editing information provided by a user; and
e2) editing the second contours of the target object on the second sectional images based on the contour editing information.

## Patentansprüche

1. Bildverarbeitungssystem, welches Folgendes aufweist:
eine 3D-Bilddatenerzeugungseinheit zum Erzeugen dreidimensionaler 3D-Bilddaten basierend auf Eingabebildsignalen;
eine Schnittbild-Extrahierungseinheit (151) zum Extrahieren erster sektionaler Bilder bzw. Schnittbilder aus den 3D-Bilddaten;
eine Konturermittlungseinheit (152) zum automatischen Ermitteln erster Konturen eines Zielobjekts auf den ersten Schnittbildern;
eine Schnittbilderzeugungseinheit (154) zum Festlegen einer Referenzebene in den 3D-Bilddaten und Schneiden des 3D-Bilds, um zweite sektionale Bilder bzw. Schnittbilder basierend auf der Referenzebene zu erzeugen, wobei die zweiten Schnittbilder parallel zu der Referenzebene sind;
eine Kontureditiereinheit (155) zum Editieren zweiter Konturen des Zielobjekts auf den zweiten Schnittbildern basierend auf Kontureditierinformationen, die von einem Benutzer zur Verfügung gestellt werden; und
eine Bilderzeugungseinheit (153) zum Erzeugen eines 3D-Volumenbilds basierend auf den ersten Konturen, die von der Konturermittlungseinheit (152) zur Verfügung gestellt werden, und den zweiten Schnittbildern, die von der Kontureditiereinheit (155) zur Verfügung gestellt werden,
wobei die Schnittbild-Extrahierungseinheit (151) Folgendes aufweist:
eine Einheit zum Festlegen einer Rotationsachse, welche durch einen Mittelpunkt der 3D-Bilddaten verläuft; und
eine Einheit zum Extrahieren der ersten Schnittbilder aus den 3D-Bilddaten in jedem definierten Winkel durch Rotieren der 3D-Bilddaten um die Rotationsachse.

2. Bildverarbeitungssystem nach Anspruch 1, wobei die Eingabebildsignale Ultraschallbildsignale sind.

3. Bildverarbeitungssystem nach Anspruch 1, welches des Weiteren eine Eingabeeinheit aufweist, um es dem Benutzer zu erlauben, Referenzebenen-Festlegungsinformationen und die Kontureditierinformationen zur Verfügung zu stellen.

4. Verfahren zur Verarbeitung eines Bilds, welches die folgenden Schritte aufweist:
a) Erzeugen von 3D-Bilddaten basierend auf Eingabebildsignalen (S110);
b) Extrahieren erster sektionaler Bilder bzw. Schnittbilder aus den 3D-Bilddaten (S130);
c) automatisches Ermitteln erster Konturen eines Zielobjekts auf den ersten Schnittbildern (S140);
d) Festlegen einer Referenzebene in den 3D-Bilddaten (S160) und Schneiden des 3D-Bilds in zweite Schnittbilder basierend auf der Referenzebene (S170), wobei die zweiten Schnittbilder parallel zu der Referenzebene sind; und
e) Editieren zweiter Konturen des Zielobjekts auf den zweiten Schnittbildern, basierend auf Kontureditierinformationen (S200), die von einem Benutzer zur Verfügung gestelltt werden; und
f) Erzeugen eines 3D-Volumenbilds basierend auf den ersten Konturen und den zweiten Schnittbildern, welche die zweiten Konturen aufweisen (S210);
wobei der Schritt b) Folgendes aufweist:
b1) Festlegen einer Rotationsachse, welche durch einen Mittelpunkt der 3D-Bilddaten verläuft; und
b2) Extrahieren der ersten Schnittbilder aus den 3D-Bilddaten in jedem definierten Winkel durch Rotieren der 3D-Bilddaten um die Rotationsachse.

5. Verfahren nach Anspruch 4, wobei die Eingabebildsignale Ultraschallbildsignale sind.

6. Verfahren nach Anspruch 4, wobei der Schritt d) Folgendes aufweist:
d1) Empfangen von Informationen zum Festlegen einer Referenzebene, die von einem Benutzer zur Verfügung gestellt werden; und
d2) Erzeugen der zweiten Schnittbilder aus den 3D-Bilddaten basierend auf den Informationen zur Festlegung der Referenzebene.

7. Verfahren nach Anspruch 4, wobei der Schritt e) Folgendes aufweist:
e1) Empfangen der Kontureditierinformationen, die von einem Benutzer zur Verfügung gestellt werden; und
e2) Editieren der zweiten Konturen des Zielobjekts auf den zweiten Schnittbildern basierend auf den Kontureditierinformationen.

## Revendications

1. Système de traitement d'image, comportant:
une unité de formation de données d'image 3D pour former des données d'image à trois dimensions 3D basée sur des signaux d'images entrantes;
une unité d'extraction d'image sectionnelle (151) pour extraire des premières images sectionnelles des données d'images 3D ;
une unité de détection de contour (152) pour détecter automatiquement des premiers contours d'un objet-cible sur les premières images sectionnelles ;
une unité de formation d'image sectionnelle (154) pour définir un plan de référence dans les données d'image 3D et trancher l'image 3D pour former des secondes images sectionnelles basées sur le plan de référence, dans lequel les secondes images sectionnelles sont parallèles au plan de référence ;
une unité d'édition de contour (155) pour éditer des seconds contours de l'objet-cible sur les secondes images sectionnelles, basées sur les informations d'édition de contour fournies par un utilisateur ; et
une unité de formation d'image (153) pour former une image de volume en 3D basée sur les premiers contours produits par l'unité de détection (152) et les secondes images sectionnelles fournies par l'unité d'édition de contour (155),
dans lequel l'unité d'extraction d'image sectionnelle (151) comporte :
une unité de détermination d'un axe de rotation passant par un centre des données de l'image 3D; et
une unité pour extraire les premières images sectionnelles des données d'images 3D sous des angles très spécifiques en faisant tourner l'image 3D autour de l'axe de rotation.

2. Système de traitement d'image selon la revendication 1, dans lequel les signaux d'image entrants sont des signaux d'image ultrasoniques.

3. Système de traitement d'image selon la revendication 1, comportant en outre une unité d'entrée pour autoriser l'utilisateur à fournir des informations déterminant un plan de référence et les informations d'édition de contour.

4. Procédé de traitement d'une image, comportant les étapes suivantes de :
a) la formation d'une image 3D basée sur des signaux d'image entrants (S110) ;
b) l'extraction d'une première image sectionnelle de données basées sur les signaux d'image 3D (S130) ;
c) la détection automatique de premiers contours de l'objet-cible sur les premières images sectionnelles (S140) ;
d) la détermination d'un plan de référence dans les données d'image 3D (S160) et le tranchage de l'image 3D en secondes images sectionnelles basées sur le plan de référence (S170), dans lequel les secondes images sectionnelles sont parallèles au plan de référence ; et
e) l'édition de contours de l'objet-cible sur les secondes images sectionnelles basées sur des informations d'édition de contour fournies par un utilisateur (S200); et
f) la formation d'une image de volume en 3D basée sur les premiers contours et les secondes images sectionnelles ayant les seconds contours (S21 0),
dans lequel l'étape b) comprend :
b1) la détermination d'un axe de rotation passant par un centre de données d'image 3D; et
b2) l'extraction des premières images sectionnelles des données d'images 3D sous des angles très spécifiques en faisant tourner l'image 3D autour de l'axe de rotation.

5. Procédé selon la revendication 4, dans lequel les signaux d'image entrants sont des signaux d'image ultrasoniques.

6. Procédé selon la revendication 4, dans lequel l'étape d) inclut :
d1) la réception des informations de détermination du plan de référence fournies par l'utilisateur ; et
d2) la formation de secondes images sectionnelles depuis les données d'images 3D, basées sur les informations de détermination du plan de référence.

7. Procédé selon la revendication 4, dans lequel l'étape e) inclut :
e1) la réception des informations d'édition de contour fournies par l'utilisateur ; et
e2) l'édition des seconds contours de l'objet-cible correspondant aux secondes images sectionnelles sur les informations d'édition de contours.
